# EUROPEAN PATENT APPLICATION

(11) **EP 0 916 682 A1**
(43) Date of publication of application: **19.05.1999**
(21) Application number: 98121641.9
(22) Date of filing: 12.11.1998
(51) Int. Cl.: C08F 230/02, A01N 57/34, A61L 2/00

(54) **Water-soluble antibacterial polymer and liquid formulation comprising it for contact lenses**

(30) Priority: 14.11.1997 JP 314021/97
(71) Applicant: Menicon Co., Ltd., Nagoya-shi Aichi-ken (JP)
(72) Inventor: Nakada, Kazuhiko, Menicon Co., Ltd., Kasugai-shi, Aichi (JP); Oono, Sadanori, Menicon Co., Ltd., Kasugai-shi, Aichi (JP)
(74) Representative: Wächtershäuser, Günter, Prof. Dr.

(57) **Abstract**

It is an object of the present invention to provide a water-soluble antibacterial polymer having an excellent antibacterial property and stability, and a liquid formulation comprising it for contact lenses, which is suitably used for disinfection or preservation of a contact lens.

A water-soluble antibacterial polymer having repeating units of the formula ( 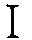 ): 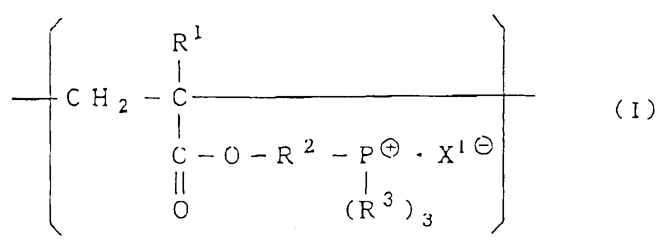 wherein R¹ is a hydrogen atom or a methyl group, R² is a C₁₋₈ alkylene group, R³ is a C₁₋₁₈ alkyl group, and X¹ is a halogen atom, and/or repeating units of the formula ( 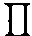 ): 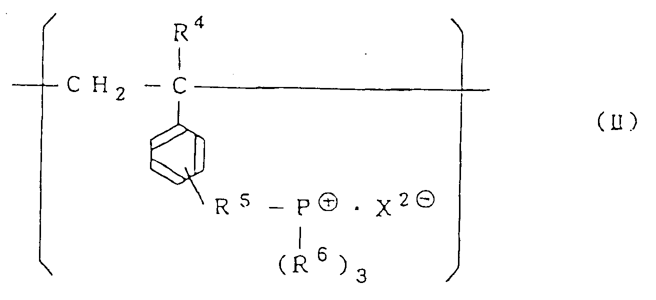 wherein R⁴ is a hydrogen atom or a methyl group, R⁵ is a C₁₋₈ alkylene group, R⁶ is a C₁₋₁₈ alkyl group, and X² is a halogen atom which is made by polymerization of a polymerizable component comprising a compound (A-1) of the formula ( 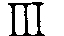 ): 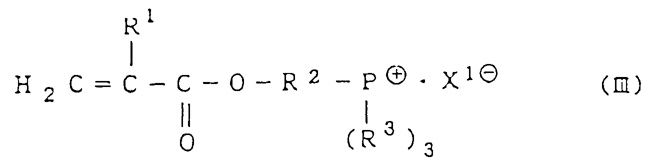 wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 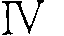 ): 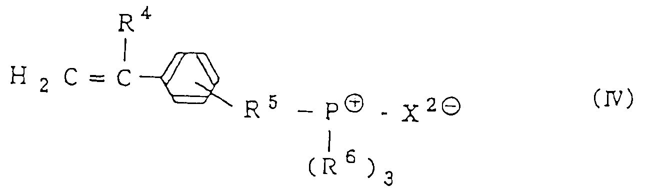 wherein R⁴ is a hydrogen atom or a methyl group, R⁵ is a C₁₋₈ alkylene group, R⁶ is a C₁₋₁₈ alkyl group, and X² is a halogen atom wherein the polymerizable component contains at least one hydrophilic monomer copolymerizable with the compound (A-1) and/or the compound (A-2).

## Description

The present invention relates to a water-soluble antibacterial polymer and a liquid formulation contain in comprising it for contact lenses. More specifically, it relates to a water-soluble antibacterial polymer having antibacterial property and stability, which is useful for a wide range of applications, and a liquid formulation for contact lenses, containing the water-soluble antibacterial polymer as a preservative or a disinfectant, which is suitably used for disinfection and preservation of contact lenses.

Usually a preservative is added in a preserving solution for contact lenses. Particularly, in water-absorptive soft contact lenses, bacteria easily breed, from the nature of their water absorptive property. Therefore, antiseptic action is required during preservation, and further periodical disinfection is rehired. Further, it has now been common to employ disinfection by a disinfectant in addition to thermal disinfection by boiling for soft contact lenses.

However, the water-absorptive soft contact lens swells when it absorbs water, and it has a problem such that the molecular interstices of the material will open, and the relatively low molecular weight preservative and disinfectant tend to be absorbed in the interstices and concentrated. Further, a problem is pointed out, such that the preservative or the disinfectant which is absorbed and concentrated in a soft contact lens, impairs eye tissues.

In order to solve the above problems, a polymer having a high molecular weight so that it is hardly to be taken into a contact lens, which also has an antibacterial effect, has been developed. For example, it is proposed to use an antibacterial quaternary ammonium group-containing polymer for disinfection of contact lenses (JP-A-6-256421).

The present invention has been made in view of the above described prior art, and it is an object of the present invention to improve the effect when the above antibacterial quaternary ammonium group-containing polymer is used for disinfection of contact lenses, and to provide a polymer highly useful for a liquid formulation, which is water soluble and excellent in antibacterial property and stability, and a liquid formulation for disinfection and preservation of contact lenses, comprising the polymer, wherein the polymer is less likely to be absorbed in the contact lenses.

The present invention provides:
1. A water-soluble antibacterial polymer having repeating units of the formula ( 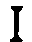 ): 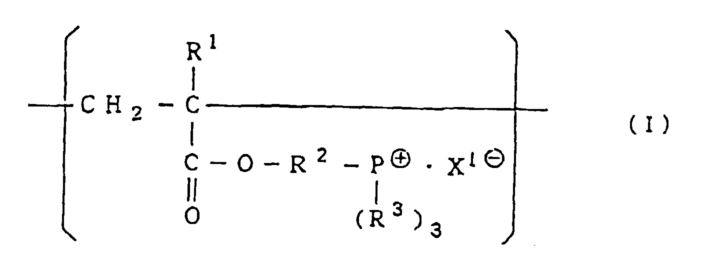 wherein R¹ is a hydrogen atom or a methyl group, R² is a C₁₋₈ alkylene group, R³ is a C₁₋₁₈ alkyl group, and X¹ is a halogen atom, and/or repeating units of the formula ( 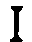 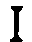 ): 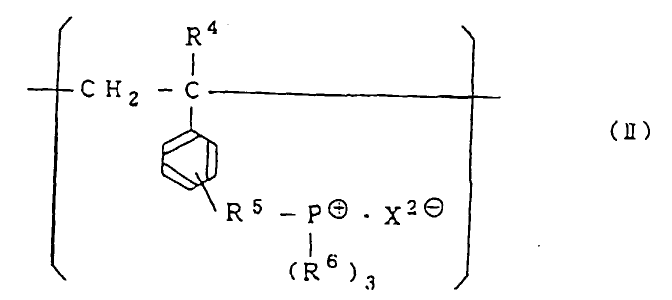 wherein R⁴ is a hydrogen atom or a methyl group, R⁵ is a C₁₋₈ alkylene group, R⁶ is a C₁₋₁₈ alkyl group, and X² is a halogen atom, which is made by polymerization of a polymerizable component comprising a compound (A-1) of the formula ( 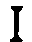 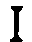 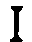 ): 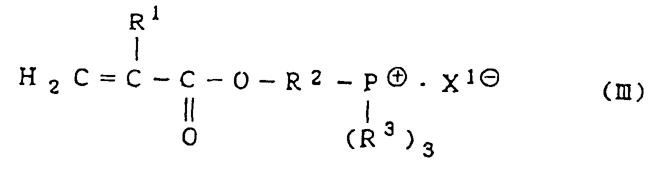 wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 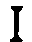 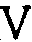 ): 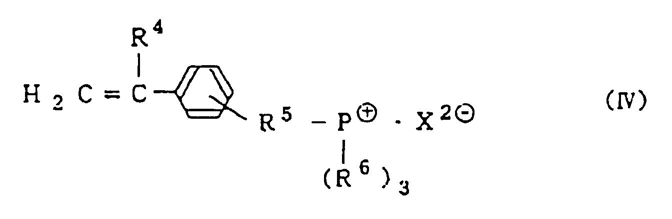 wherein R⁴ is a hydrogen atom or a methyl group, R⁵ is a C₁₋₈ alkylene group, R⁶ is a C₁₋₁₈ alkyl group, and X² is a halogen atom, wherein the polymerizable component contains at least one hydrophilic monomer copolymerizable with the compound (A-1) and/or the compound (A-2), and
2. A liquid formulation for contact lenses comprising the water-soluble antibacterial polymer.

As mentioned above, the water-soluble antibacterial polymer of the present invention is a polymer having repeating units of the formula ( 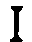 ): 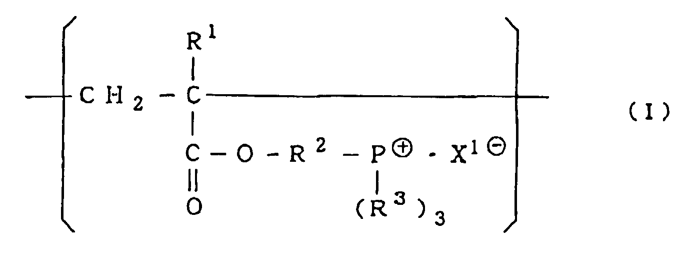 wherein R¹ is a hydrogen atom or a methyl group, R² is a C₁₋₈ alkylene group, R³ is a C₁₋₁₈ alkyl group, and X¹ is a halogen atom and/or repeating units of the formula ( 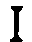 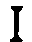 ): 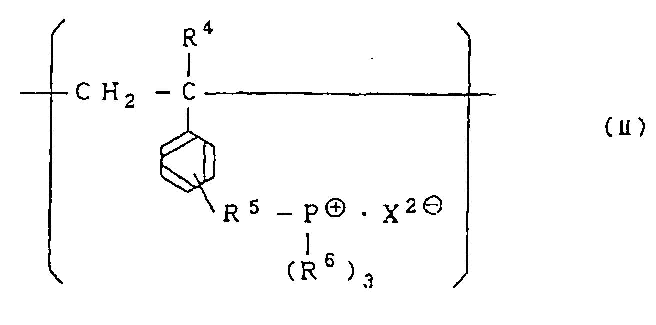 wherein R⁴ is a hydrogen atom or a methyl group, R⁵ is a C₁₋₈ alkylene group, R⁶ is a C₁₋₁₈ alkyl group, and X² is a halogen atom, which is made by polymerization of a polymerizable component comprising a compound (A-1) of the formula ( 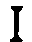 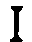 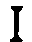 ): 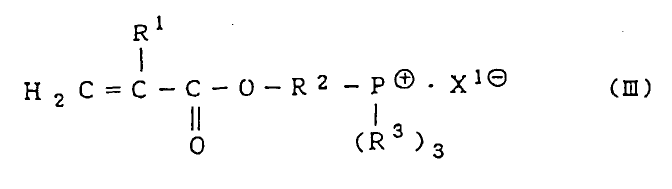 wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 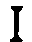 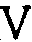 ): 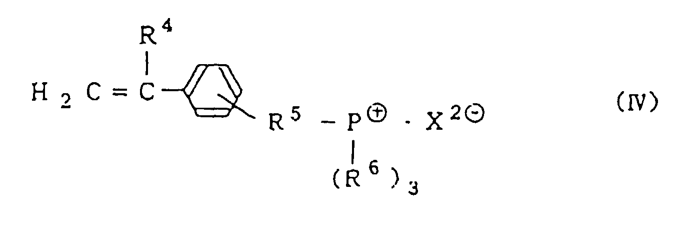 wherein R⁴ is a hydrogen atom or a methyl group, R⁵ is a C₁₋₈ alkylene group, R⁶ is a C₁₋₁₈ alkyl group, and X² is a halogen atom, wherein the polymerizable component contains at least one hydrophilic monomer copolymerizable with the compound (A-1) and/or the compound (A-2) and it is a component showing an antibacterial effect.

The reason why the water-soluble antibacterial polymer has an excellent antibacterial property is that quaternary phosphonium groups exist in the repeating units of the formula ( 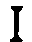 ) and the repeating units of the formula ( 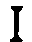 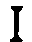 ), and further the antibacterial polymer is excellent in stability, and highly water-soluble.

As the water-soluble antibacterial polymer of the present invention, preferred is a polymer (hereinafter referred to as polymer (A)) made by polymerization of a polymerizable component comprising a compound (A-1) of the formula ( 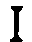 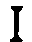 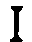 ): 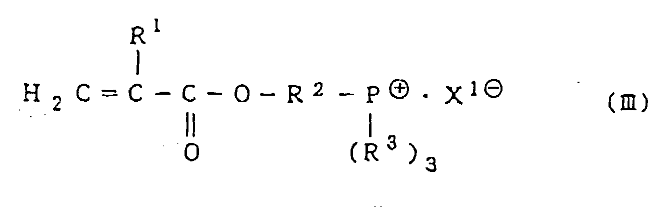 wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 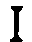 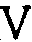 ): 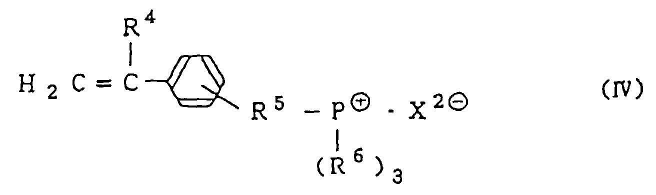 wherein R⁴ is hydrogen atom or a methyl group, R⁵ is a C₁₋₈ alkylene group, R⁶ is a C₁₋₁₈ alkyl group, and X² is a halogen atom.

Each of the compound (A-1) and the compound (A-2) is a component showing an antibacterial effect, by the quaternary phosphonium groups which exist in the molecule.

In the formula ( 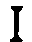 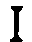 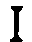 ) representing the compound (A-1) and the formula ( 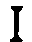 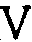 ) representing the compound (A-2), each of R² and R⁵ is a C₁₋₈ alkylene group. However, from the viewpoint of keeping the water solubility significantly, each of R² and R⁵ is preferably a C₁₋₄ alkylene group. And each of R³ and R⁶ is a C₁₋₁₈ alkyl group, but, from the viewpoint of showing a high water solubility and antibacterial property, each of R³ and R⁶ is preferably a C₁₋₁₂ alkyl group. The three R³ in the formula ( 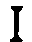 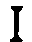 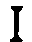 ) may be same or different, and the three R⁶ in the formula ( 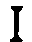 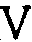 ) may be same or different.

Each of X¹ in the formula ( 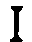 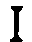 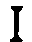 ) and X² in the formula ( 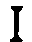 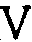 ) is a halogen atom, such as a chlorine atom, a bromine atom or an iodine atom.

Specific examples of the compound (A-1) include tri-n-butyl(2-(meth)acryloyloxyethyl)phosphonium chloride, tri-n-hexyl(2-(meth)acryloyloxyethyl)phosphonium chloride, tri-n-octyl(2-(meth)acryloyloxyethyl)phosphonium chloride, tri-n-butyl(2-(meth)acryloyloxyethyl)phosphonium bromide, tri-n-hexyl(2-(meth)acryloyloxyethyl)phosphonium bromide, tri-n-octyl(2-(meth)acryloyloxyethyl)phosphonium bromide, tri-n-butyl(2-(meth)acryloyloxyethyl)phosphonium iodide, tri-n-hexyl(2-(meth)acryloyloxyethyl)phosphonium iodide and tri-n-octyl(2-(meth)acryloyloxyethyl)phosphonium iodide.

Specific examples of the compound (A-2) include tri-n-butyl(4-vinylbenzyl)phosphonium chloride, tri-n-hexyl(4-vinylbenzyl)phosphonium chloride, tri-n-octyl(4-vinylbenzyl)phosphonium chloride, tri-n-butyl(4-vinylbenzyl)phosphonium bromide, tri-n-hexyl(4-vinylbenzyl)phosphonium bromide, tri-n-octyl(4-vinylbenzyl)phosphonium bromide, tri-n-butyl(4-vinylbenzyl)phosphonium iodide, tri-n-hexyl(4-vinylbenzyl)phosphonium iodide and tri-n-octyl(4-vinylbenzyl)phosphonium iodide.

The compound (A-1) and the compound (A-2) may be used alone or in combination as a mixture of two or more of them. Among these, tri-n-butyl(2-methacryloyloxyethyl)phosphonium chloride and tri-n-butyl (4-vinylbenzyl)phosphonium chloride are preferred, since they give water solubility significantly to the obtained polymer.

It is preferred to suitably adjust the amounts of the compound (A-1) and/or the compound (A-2) and the hydrophilic monomer in the polymerizable component, taking the resulting antibacterial property into consideration, and in addition, considering the amount of another polymerizable monomer described hereinafter.

To obtain the polymer (A), it is necessary to incorporate at least one hydrophilic monomer copolymerizable with the compound (A-1) and/or the compound (A-2), in order to improve the water solubility of the obtained polymer (A).

The hydrophilic monomer may, for example, be an alkyl(meth)acrylamide, such as N,N-dimethyl (meth)acrylamide, N,N-diethyl(meth)acrylamide, N,N-dipropyl(meth)acrylamide or N,N-dibutyl(meth)acrylamide, an N-vinyl lactam such as N-vinyl-2-pyrrolidone, N-vinylpyrrolidone or N-vinylcaprolatctom, a hydroxyalkyl (meth)acrylate such as 2-hydroxyethyl (meth)acrylate, hydroxypropyl (meth)acrylate, hydroxybutyl (meth)acrylate or hydroxyoentyl (meth)acrylate. They may be used alone or in combination as a mixture of two or more of them. Among these, N,N-dimethylacrylamide and N-vinyl-2-pyrrolidone are preferred, from the viewpoint that the water solubility of the obtained polymer (A) increases, the polymer (A) will have a suitable viscosity, and further, when the polymer (A) is used for the liquid formulation for contact lenses, the transparency of the liquid formulation for contact lenses improves.

In the case where the hydrophilic monomer is used as a polymerizable component, the ratio of the compound (A-1) and/or the compound (A-2) to the hydrophilic monomer, i.e. the weight ratio of (the compound (A-1) and/or the compound (A-2))/(hydrophilic monomer) is at least 5/100, preferably 10/100, in order to obtain an adequate effect for imparting the antibacterial property by using the compound (A-1) and/or the compound (A-2). And in order to adequately obtain the effect of improving the water solubility by using the hydrophilic monomer, it is preferably at most 100/50, particularly preferably at most 100/80.

To obtain the polymer (A), the entire amount of the polymerizable component is the compound (A-1) and/or the compound (A-2) and the hydrophilic monomer. However, in the present invention, another polymerizable monomer which is copolymerizable with the compound (A-1) and/or the compound (A-2) and the hydrophilic monomer may be incorporated, within a range not to impair the purpose of the present invention.

Another polymerizable monomer may, for example, be a linear, branched or cyclic alkyl (meth)acrylate such as methyl (meth)acrylate, ethyl (meth)acrylate, isopropyl (meth)acrylate, n-propyl (meth)acrylate, isobutyl (meth)acrylate, n-butyl (meth)acrylate, 2-ethylhexyl (meth)acrylate, n-octyl (meth)acrylate, n-decyl (meth)acrylate, n-dodecyl (meth)acrylate, t-butyl (meth)acrylate, pentyl (meth)acrylate, t-pentyl (meth)acrylate, hexyl (meth)acrylate, heptyl (meth)acrylate, nonyl (meth)acrylate, stearyl (meth)acrylate, cyclopentyl (meth)acrylate or cyclohexyl (meth)acrylate; styrene; α-methylstyrene; an alkylstyrene such as methylstyrene, ethyl styrene, propylstyrene, butylstyrene, t-butylstyrene, isobutylstyrene or pentylstyrene; or an alkyl-α-methylstyrene such as methyl-α-methylstyrene, ethyl-α-methylstyrene, propyl-α-methylstyrene, butyl-α-methylstyrene, t-butyl-α-methylstyrene, isobutyl-α-methylstyrene or pentyl-α-methylstyrene.

Such polymerizable polymers as mentioned above may be used alone or in combination as a mixture of two or more of them. It is possible to suitably adjust the amount of such another polymerizable monomer, depending on the amount of the compound (A-1) and/or the compound (A-2) and the hydrophilic monomer, so that the amount of such another polymerizable monomer will be within the range of at most 10 wt%, i.e. the amount of the compound (A-1) and/or the compound (A-2) and the hydrophilic monomer is at least 90 wt%, to make the total amount of the polymerizable component 100 wt%.

In the present invention, the polymer (A) which is one example of the water-soluble antibacterial polymer, can be obtained, for example, by adjusting the polymerizable component comprising the compound (A-1) and/or the compound (A-2), the hydrophilic monomer and another hydrophilic monomer, within the range of the amounts defined above, adding a radical polymerization initiator thereto, and polymerizing them by a usual method.

The usual method may, for example, be a method wherein a radical polymerization initiator is added, and the mixture is heated at room temperature, or, for example, in a case where a solvent is used, at a temperature lower than the boiling point of the solvent; or a method wherein electromagnetic waves such as microwaves, ultraviolet lights, or radiations (γ rays) are irradiated to conduct polymerization. When the heat polymerization is conducted, the temperature may be raised stepwise. The polymerization may be conducted by a block polymerisation method or a solution polymerization method by using a solvent such as tetrahydrofuran, an alcohol, toluene or dimethylformamide, or may be conducted by another method.

The radical polymerization initiator may, for example, be azobisisobutyronitrile, azobisdimethyl valeronitrile, benzoyl peroxide, t-butyl hydroperoxide or cumene hydroperoxide. These may be used alone or in combination as a mixture of two or more of them. When light rays are utilized for polymerization, it is preferred to further add a photo-polymerization initiator or a sensitizer. The amount of the polymerization initiator or the sensitizer is preferably from about 0.001 to about 2 parts ("parts" means parts by weight, and the same applies hereinafter), particularly preferably from about 0.01 to about 1 part, based on the total 100 parts of the polymerizable component.

A polymer such as the polymer (A) thus obtained is purified by washing with e.g. water, methanol or a mixed solution of them, followed by deairing drying, to obtain the water-soluble antibacterial polymer of the present invention.

The water solubility of the water-soluble antibacterial polymer means that the polymer is soluble in water in an amount sufficient to be effective in antibacterial property.

When it is used, for example, for liquid formulation for contact lenses, particularly for liquid formulation for water-absorptive soft contact lenses, as described hereinafter, the weight average molecular weight of the water-soluble antibacterial polymer of the present invention is preferably at least 2,000, more preferably at least 3,000, in order to eliminate the possibility that it is taken into the contact lens. Further, it is preferably at most 1,000,000, more preferably at most 800,000, in order to avoid a problem such that the viscosity of the liquid formulation for contact lenses tends to be so high that handling will be difficult during the production. The weight average molecular weight of the water-soluble antibacterial polymer is a value measured by using the gel permeation chromatography (hereinafter referred to as GPC).

The water-soluble antibacterial polymer of the present invention is one having repeating units of the formula ( 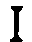 ): 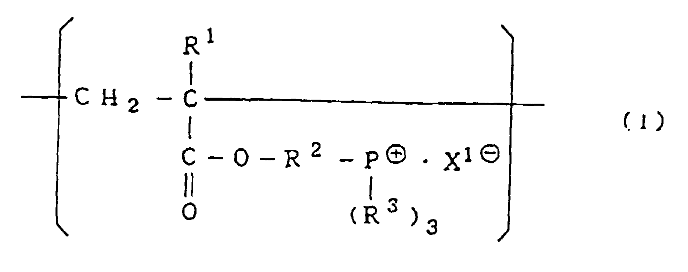 wherein R¹ is a hydrogen atom or a methyl group, R² is a C₁₋₈ alkylene group, R³ is a C₁₋₁₈ alkyl group, and X¹ is a halogen atom, and/or repeating units of the formula ( 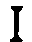 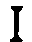 ): 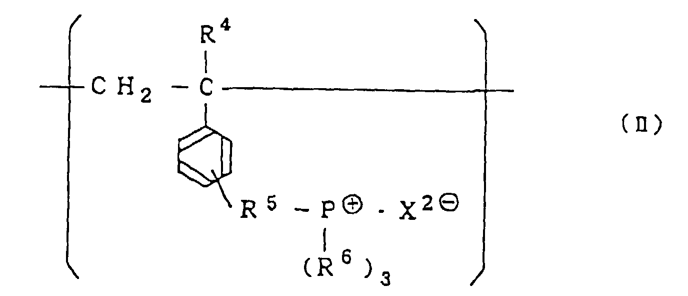 wherein R⁴ is a hydrogen atom or a methyl group, R⁵ is a C₁₋₈ alkylene group, R⁶ is a C₁₋₁₈ alkyl group, and X² is a halogen atom, which is made by polymerization of a polymerizable component comprising a compound (A-1) of the formula ( 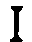 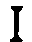 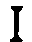 ): 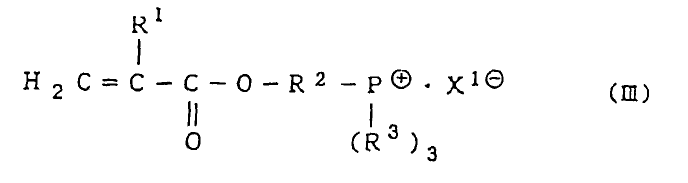 wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 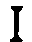 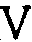 ): 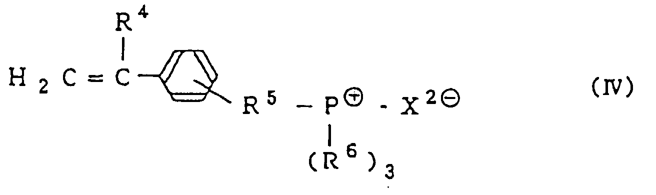 wherein R⁴ is a hydrogen atom or a methyl group, R⁵ is a C₁₋₈ alkylene group, R⁶ is a C₁₋₁₈ alkyl group, and X² is a halogen atom wherein the polymerizable component contains at least one hydrophilic monomer copolymerizable with the compound (A-1) and/or the compound (A-2).

In the water-soluble antibacterial polymer of the present invention, either the repeating units of the formula ( 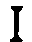 ) or the repeating units of the formula ( 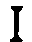 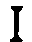 ) may be included or both of them may be included. In a case where both of them are included, they may be in a random or block arrangement.

The preferred modes of R², R³ and X¹ in the formula ( 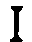 ) and R⁵, R⁶ and X² in the formula ( 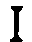 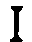 ) are the same, for example, as R², R³ and X¹ in the formula ( 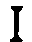 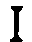 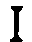 ) representing the formula (A-1) and R⁵, R⁶ and X² in the formula ( 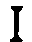 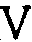 ) representing the formula (A-2), respectively.

The liquid formulation for contact lenses of the present invention is one containing the water-soluble antibacterial polymer.

The content of the water-soluble antibacterial polymer in the liquid formulation for contact lenses is preferably at least 0.0001 w/v%, particularly preferably at least 0.001 w/v%, in order to obtain an adequate antiseptic effect or a disinfecting effect by the water-soluble antibacterial polymer. And, it is preferably at most 10 w/v%, particularly preferably at most 5 w/v%, for handling efficiency.

In the liquid formulation for contact lenses of the present invention, a compounding agent which is commonly used for a disinfectant of contact lenses, such as a chelating agent, a buffer or an isotonicity agent may be contained in addition to the water-soluble antibacterial polymer.

The chelating agent has a characteristic to keep the liquid formulation from depositing on a contact lens.

The chelating agent is not particularly limited so long as it is ophthalmologically acceptable, and the examples of which include ethylenediaminetetraacetic acid and its sodium salt, phytic acid and citric acid.

The content of the chelating agent in the liquid formulation for contact lenses is preferably at least 0.001 mol/ℓ, particularly preferably at least 0.0015 mol/ℓ, in order to adequately obtain the effect of preventing deposition of the liquid formulation on a contact lens. And, it is preferably at most 0.1 mol/ℓ, particularly preferably at most 0.05 mol/ℓ, since if the content of the chelating agent is too much, the improvement in the effect of incorporating it is small, and such is uneconomical.

The buffer serves to adjust the pH of the obtained liquid formulation for contact lenses constant within a range of from about 5 to about 9 which is close to the tear.

The buffer is not particularly limited so long as it is ophthalmologically acceptable, and examples of which include boric acid and its sodium salt, phosphoric acid and its sodium salt, citric acid and its sodium salt, lactic acid and its sodium salt, an amino acid such as glycine or glutamic acid and its sodium salt, and malic acid and its sodium salt.

The content of the buffer in the liquid formulation for contact lenses is preferably at least 0.005 mol/ℓ, particularly preferably at least 0.01 mol/ℓ, in order to obtain an adequate buffer effect. And, it is preferably at most 0.5 mol/ℓ, particularly preferably at most 0.15 mol/ℓ, since if the content of the buffer is too much, the effect of buffer no longer increase, and the osmotic pressure may be increased so that it tends to influence the form of a contact lens.

The isotonicity agent serves to bring the osmotic pressure of the obtained liquid formulation for contact lenses close to the osmotic pressure of the tear (from 280 to 300 mOs/kg).

The isotonicity agent is not particularly limited so long as it is ophthalmologically acceptable, and examples of which include an inorganic salt such as sodium chloride, potassium chloride or calcium chloride and the above-mentioned buffer.

The content of the isotonicity agent in a liquid formulation for contact lenses is preferably at least 0.01 mol/ℓ, particularly preferably at least 0.05 mol/, in order to obtain an adequate osmotic pressure. And, it is preferably at most 0.5 mol/ℓ, particularly preferably at most 0.15 mol/ℓ, since if the content of the isotonicity agent is too much, the osmotic pressure becomes high and the buffer tends to influence the form of a contact lens.

The chelating agents, the buffers or the isotonicity agents may be used alone or in combination as a mixture of two or more of them, respectively.

As mentioned above, the liquid formulation for contact lenses of the present invention contains, the water-soluble antibacterial polymer as an active ingredient, and as the case requires, it contains another component such as a compounding agent, e.g. chelating agent, a buffer or an isotonicity agent. As a medium, water such as distilled water or purified water may be used. Such an aqueous medium e.g. water may be used to bring the total of the liquid formulation for contact lenses to 100%.

The liquid formulation for contact lenses of the present invention is prepared, for example, by adding the water-soluble antibacterial polymer into the predetermined amount of the aqueous medium, then adding a compounding agent, such as a chelating agent, a buffer or an isotonicity agent, if necessary, mixing and stirring them well to dissolve them, followed by filtration.

By soaking contact lenses in the liquid formulation for contact lenses of the present invention thus obtained, various types of contact lenses can be preserved or disinfected without impairing their forms.

Now, the water-soluble antibacterial polymer of the present invention and the liquid formulation for contact lenses made of it will be described in further detail with reference to Examples. However, it should be understood that the present invention is by no means restricted to such specific Examples.

### EXMAPLE 1

20 g of tetrahydrofuran, 5 g of tri-n-octyl(4-vinylbenzyl)phosphonium chloride, 5 g of N-vinyl-2-pyrrolidone and 0.01 g of azobisisobutyronitrile were charged into a three necked distillation flask equipped with a stirrer and a Dimroth condenser, and stirred for six hours at a temperature of 50°C in a water bath. The reaction solution was deaired and dried and the obtained gel was washed with a mixed solution of water and methanol, deaired and dried again, to obtain a polymer. The amount obtained was 4 g, and the yield was 40%, based on tri-n-octyl(4-vinylbenzyl)phosphonium chloride and N-vinyl-2-pyrrolidone.

The infrared absorption spectrum of the polymer was measured by using FT-IR 8,300 manufactured by Nippon Bunko, K.K. and a KBr tablet.

As a result, the absorption attributable to the C=C double bond at 1637 cm⁻¹ disappeared, and the polymerization was confirmed.

It was confirmed from the results that the obtained polymer has repeating units of the formula: 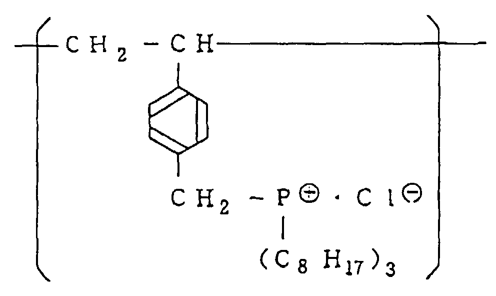 and repeating units of the formula:

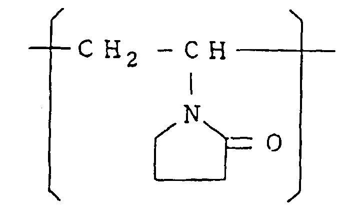

The weight average molecular weight of the polymer measured by GPC (eluant: 100 mM boric acid, pH 9.1) was about 30,000.

### EXAMPLE 2

The polymer obtained in Example 1 was added to distilled water, and the mixture was stirred at room temperature to dissolve the polymer, whereby a liquid formulation having the polymer content of 200 ppm (by weight) was prepared.

The liquid formulation was subjected to antiseptic effect test by organism challenge test according to the United States Pharmacopoeia.

1 × 10⁶ cells of Gram positive vegetative bacteria (Staphylococcus aureus) and Gram negative vegetative bacteria (Escherichia coli) were inoculated to the prepared liquid formulation as a test sample. 24 hours later, the numbers of the Gram positive bacteria and the Gram negative bacteria were found to have decreased to 1 × 10⁴ cells and 1 × 10⁴ cells, respectively.

As a result, the liquid formulation was found to have an excellent antibacterial property imparted by the polymer obtained in Example 1.

The water solubility of the polymer was confirmed by the fact that the 200 ppm liquid formulation of the polymer obtained in Example 1 had such an antibacterial effect, and the solubility of the polymer in water at a temperature of 20°C was at least 1 g.

The water-soluble antibacterial polymer of the present invention, having an excellent antibacterial property, which is less likely to decompose, stable and water soluble, is highly useful as a liquid formulation.

Therefore, the liquid formulation for contact lenses of the present invention obtained by using the water-soluble antibacterial polymer, wherein the water-soluble antibacterial polymer is less likely to be absorbed in a contact lens, is suitably used for disinfection or preservation of a contact lens.

## Claims

1. A water-soluble antibacterial polymer having repeating units of the formula ( 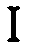 wherein R¹ is a hydrogen atom or a methyl group, R² is a C₁₋₈ alkylene group, R³ is a C₁₋₁₈ alkyl group, and X¹ is a halogen atom, and/or repeating units of the formula ( 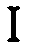 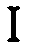 ): 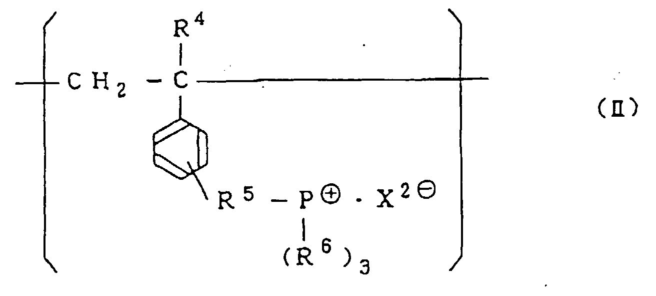 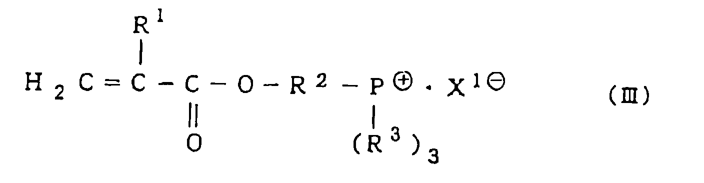 wherein R⁴ is a hydrogen atom or a methyl group, R⁵ is a C₁₋₈ alkylene group, R⁶ is a C₁₋₁₈ alkyl group, and X² is a halogen atom, which is made by polymerization of a polymerizable component comprising a compound (A-1) of the formula ( 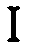 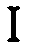 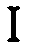 ): wherein R¹ is a hydrogen atom or a methyl group, R² is a C₁₋₈ alkylene group, R³ is a C₁₋₁₈ alkyl group, and X¹ is a halogen atom, and/or a compound (A-2) of the formula ( 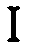 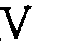 ): 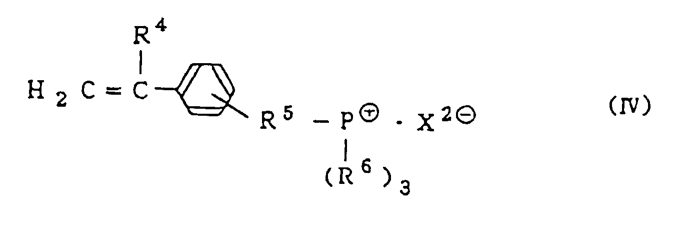 wherein R⁴ is a hydrogen atom or a methyl group, R⁵ is a C₁₋₈ alkylene group, R⁶ is a C₁₋₁₈ alkyl group, and X² is a halogen atom, wherein the polymerizable component contains at least one hydrophilic monomer copolymerizable with the compound (A-1) and/or the compound (A-2).

2. The water-soluble antibacterial polymer according to Claim 1, wherein the ratio of the compound (A-1) and/or the compound (A-2) to the hydrophilic monomer, i.e. the weight ratio of (the compound (A-1) and/or the compound (A-2))/(hydrophilic monomer), is at least 5/100.

3. The water-soluble antibacterial polymer according to Claim 1 or 2, which has a weight average molecular weight of from 2,000 to 1,000,000.

4. A liquid formulation for contact lenses comprising the water-soluble antibacterial polymer as defined in Claims 1, 2 or 3.
